# EUROPÄISCHE PATENTSCHRIFT

(11) **EP 3 589 612 B1**
(45) Veröffentlichungstag und Bekanntmachung des Hinweises auf die Patenterteilung: **31.03.2021**
(21) Anmeldenummer: 18706515.6
(22) Anmeldetag: 22.02.2018
(51) Int. Cl.: C07C 253/20, C07C 255/10, C07D 257/04

(54) **VERFAHREN ZUR HERSTELLUNG VON FLUORALKYLNITRILEN UND DEN ENTSPRECHENDEN FLUORALKYLTETRAZOLEN**
PROCESSES FOR THE PREPARATION OF FLUORALKYL NITRILES AND THE CORRESPONDING FLUORALKYL TETRAZOLES
PROCÉDÉS DE PRÉPARATION DES NITRILES FLUORALKYLES ET DES TÉTRAZOLES FLUORALKYLES CORRESPONDANTS

(30) Priorität: 28.02.2017 EP 17158376
(43) Veröffentlichungstag der Anmeldung: 08.01.2020
(73) Patentinhaber: Bayer CropScience Aktiengesellschaft, 40789 Monheim am Rhein (DE)
(72) Erfinder: SCHNATTERER, Albert, 51373 Leverkusen (DE); VERMEHREN, Jan, 65510 Idstein (DE); BECKMANN, Edith, 50829 Köln (DE); HÄSELHOFF, Claus, Christian, 45968 Gladbeck (DE); HAMMERER, Tim, 52499 Baesweiler (DE); GRASSER, Stefan, 51377 Leverkusen (DE)
(74) Vertreter: BIP Patents
(86) Internationale Anmeldenummer: PCT/EP2018/054385
(87) Internationale Veröffentlichungsnummer: WO 2018/158131

(56) Entgegenhaltungen:
- CRAWFORD M J ET AL: "Synthesis and characterization of perfluorinated nitriles and the corresponding sodium 5-perfluoroalkyltetrazolate salts", JOURNAL OF FLUORINE CHEMISTRY, ELSEVIER, NL, Bd. 129, Nr. 12, 1. Dezember 2008 (2008-12-01), Seiten 1199-1205, XP025678338, ISSN: 0022-1139, DOI: 10.1016/J.JFLUCHEM.2008.09.007 [gefunden am 2008-09-25] in der Anmeldung erwähnt
- FERNANDEZ ET AL.: "Synthesis of acetonitrile by dehydration of acetamide on an active ZnO catalyst: A comparison with zeolite catalysts", INDIAN JOURNAL OF CHEMICAL TECHNOLOGY, Bd. 5, Nr. 6, November 1998 (1998-11), Seiten 405-406, XP002768858, in der Anmeldung erwähnt

## Beschreibung

DE3600811A1 offenbart die Herstellung von Nitrilen durch katalytische Dehydratisierung von Carbonsäureamiden an zeolithischen Katalysatoren in Gegenwart von Ammoniak. Die Publikation beschreibt nicht die Verwendung von Fluoralkylacetamiden als Edukte.

Bei einigen der im Stand der Technik beschriebenen Verfahren müssen wasserabspaltende Reagenzien (wie z.B. Trifluoressigsäureanhydrid) in mehrfachem Überschuss eingesetzt werden. Ein Recycling der eingesetzten Stoffe und Lösungsmittel ist notwendig. Bei Verwendung von Phosphorpentoxid kommt es bei dieser Art der Reaktionen in der Regel zu nicht mehr rührbaren, erstarrten Mischungen, die einer großtechnischen Realisierung entgegenstehen.

Keine der im Stand der Technik beschriebenen Verfahren beschreibt die Dehydratisierung von fluorierte Carbonsäureamiden mit Katalysatoren wie beispielsweise Zeolithen oder Aluminiumphosphaten. Der Einsatz solcher Katalysatoren hat den Vorteil, dass diese umweltfreundlichen und preisgünstig sind und für die weitere Herstellung von Fluoralkylnitrilen (z.B. in einem kontinuierlichen Prozess) wieder eingesetzt werden können. Bisher ging der Fachmann davon aus, dass die Dehydratisierung von fluorierten Carbonsäureamiden mit solchen Katalysatoren auf Grund der Reaktivität der Fluorreste nicht zu gewünschten Ergebnissen führt. Dies wird durch die vorliegende Erfindung widerlegt.

Ausgehend vom Stand der Technik ergibt sich als Aufgabe der vorliegenden Erfindung, ein möglichst effizientes, selektives und kostengünstiges Verfahren zur Herstellung von fluorierten Alkylnitrilen bzw. den daraus erhältlichen fluorierten Alkyltetrazolen bereitzustellen, welches vorzugsweise einfach durchzuführen ist. Die mit diesem angestrebten Verfahren erhältlichen fluorierten Alkylnitrile bzw. fluorierten Alkyltetrazole sollen dabei vorzugsweise mit hoher Selektivität und hoher Ausbeute erhalten werden. Insbesondere soll das angestrebte Verfahren den Erhalt der gewünschten Zielverbindungen ohne die Notwendigkeit komplexer Aufreinigungsmethoden ermöglichen.

Die Aufgabe wurde gemäß der vorliegenden Erfindung gelöst durch Verfahren zum Herstellen von Fluoralkylnitrilen der allgemeinen Formel (I) durch katalytische Gasphasen-Dehydratisierung, 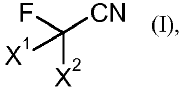 in welcher
X¹ und X² unabhängig voneinander für ein Halogen, Wasserstoff, Methyl stehen,
dadurch gekennzeichnet, dass man
fluorierte Carbonsäureamide der Formel (II) 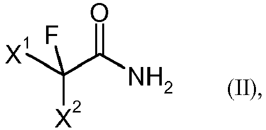 in welcher X¹ und X² die oben genannten Bedeutungen haben, in die Gasphase überführt und in Gegenwart von Katalysatoren ausgewählt aus der Gruppe von Zeolithen, Aluminiumphosphat, Zirkondioxid und Heteropolysäuren (vorzugsweise ausgewählt aus der Gruppe von Zeolithen und Aluminiumphosphat und noch bevorzugter ausgewählt aus der Gruppe der Zeolithe)
   umsetzt.
X¹ und X² stehen dabei unabhängig voneinander bevorzugt für Fluor, Chlor, Wasserstoff, Methyl und noch bevorzugter nur für Fluor.

In einer weiteren bevorzugten Ausführungsform der Erfindung werden die Fluoralkylnitrile der allgemeinen Formel (I), 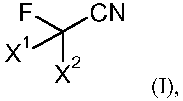 nach Herstellung mit dem oben beschriebenen Verfahren mit Natriumazid in Gegenwart eines Lösungsmittels direkt zu den entsprechenden Fluoralkyltetrazolen der allgemeinen Formel (III) 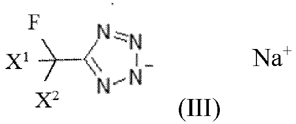 in welcher X¹ und X² die oben genannten Bedeutungen haben,
umgesetzt.

Das Fluoralkyltetrazol der Formel (III) liegt dabei vorzugsweise in der Form des Na-Salzes vor.

Die gemäß der vorliegenden Erfindung verwendeten fluorierten Alkylamide der Formel (II) sind kommerziell erhältlich oder können nach literaturbekannten Verfahren leicht hergestellt werden (WO 03/080563).

Die Herstellung der Fluoralkyltetrazole aus den Fluoralkylnitrilen ist beispielsweise auch im Journal of Fluorine Chemistry 120 (2008) 1199-1205 beschrieben worden.

Überraschenderweise lassen sich die fluorierten Alkylnitrile der Formel (I) bzw. daraus entsprechend hergestellten Fluoralkyltetrazole der allgemeinen Formel (III) unter den erfindungsgemäßen Bedingungen sicher und mit guten Selektivitäten und Ausbeuten in hoher Reinheit herstellen. Dabei weist das erfindungsgemäße Verfahren die im Zusammenhang mit dem Stand der Technik beschriebenen Nachteile nicht auf.

Im Zusammenhang mit der vorliegenden Erfindung umfasst der Begriff Halogene bzw. Halogenide, soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

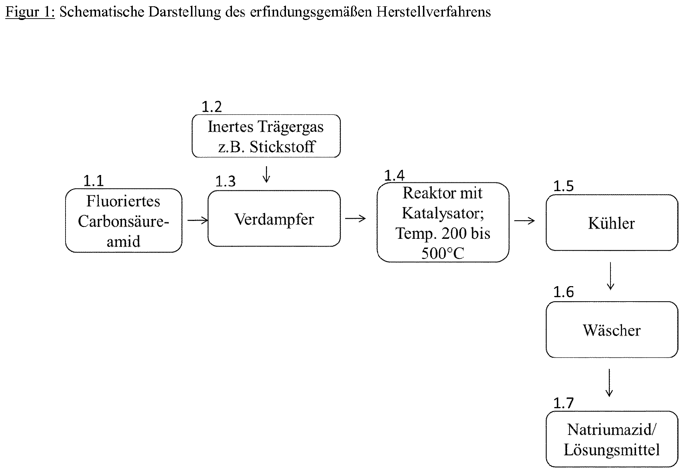

### Gasphasen-Dehydratisierung (Schritte 1.1 bis 1.5 in Figur 1)

Zunächst wird ein Reaktor mit einem erfindungsgemäß beschriebenen Katalysator (1.4 in Figur 1) befüllt und optional mit Stickstoff inertisiert. Danach wird die gewünschte Reaktortemperatur eingestellt (vorzugsweise zwischen 200-500°C und noch bevorzugter zwischen 250-350°C). Anschließend wird auf das Reaktionsgas (1.2 in Figur 1) fluorierte Carbonsäureamid der Formel (II) (vorzugsweise in Kombination mit einem inerten Trägergas wie Stickstoff) umgeschaltet. Das bei Raumtemperatur fest vorliegende fluorierte Carbonsäureamid der Formel (II) wird vorzugsweise (in einer beheizten Kolbenpumpe) aufgeschmolzen (1.1 in Figur 1) und über eine beheizte Leitung einem vorgeschalteten Verdampfer (1.3 in Figur 1) zugeführt, in welchem es in den gasförmigen Zustand überführt wird. Die Temperatur im Verdampfer ist dabei vorzugsweise zwischen 200 und 300°C. Wenn das fluorierte Carbonsäureamid der Formel (II) bereits flüssig vorliegt, wird es direkt über eine Pumpe dem Verdampfer zugeführt. Für die Menge an Amid gibt es in der Regel keine Beschränkung. Technisch sinnvoll ist eine Katalysatorbelastung die weder einen zu hohen Druckverlust noch eine zu schnelle Katalysatordesaktivierung noch eine unvollständige Reaktion hervorruft. Nebenprodukte bzw. nicht umgesetzte Edukte werden durch einen Kühler (1.5 in Figur 1) bei Temperaturen von vorzugsweise zwischen 1 und 10°C auskondensiert.

### Katalysatoren

Zeolithe sind kristalline Alumosilikate, die in zahlreichen Modifikationen in der Natur vorkommen, aber auch synthetisch hergestellt werden können und u.a. als Katalysatoren eingesetzt werden können.

Zeolithe können durch die folgende Summenformel beschrieben werden:

Mⁿ⁺_{x/n}[(AlO₂)⁻ₓ(SiO₂)_{y}]·z H₂O

Hierin ist M typischerweise ein Kation eines Alkali- oder Erdalkalimetalls oder ein AmmoniumIon, das für den elektrischen Ladungsausgleich der negativ geladenen Aluminium-Sauerstoff-Tetraeder benötigt wird. "n" ist die Ladung des Kations, die typischerweise 1 oder 2 ist. Das molare Verhältnis von SiO₂ zu AlO₂ bzw. y/x in der Summenformel wird als Modul bezeichnet. Der Buchstabe "z" gibt die Anzahl der vom Kristall aufgenommenen Wassermoleküle an.

Durch mehrere Experimente wurde untermauert, dass ein für die Herstellung von Fluoralkylnitrilen besonders geeigneter aktiver Zeolith-Katalysator in der Protonen-Form, d.h. M = H und n = 1 vorliegen sollte. Je nach Zeolith wird ab etwa 250 °C Wasser aus dem Kristall freigesetzt, so dass z von den genauen Reaktionsbedingungen abhängt. Das molare Verhältnis y/x hat Einfluss auf die Anzahl der Lewis-sauren Al-Zentren und auf deren relative Stärke untereinander. Es ist in weiten Bereichen variierbar. Bevorzugt sind molare Verhältnisse von 10 bis 120, besonders bevorzugt von 20 bis 100.

Zeolithe in der Protonen-Form (H-Form) können auch zu Beginn des Prozesses (quasi *in situ*) innerhalb des Reaktors hergestellt werden. Hierzu sind alle Kationen geeignet, die bei höheren Temperaturen (> 150 °C) derart zerfallen, dass sich eine flüchtige Komponente und ein Proton bilden. Bevorzugt sind daher auch Ammonium-Kationen der allgemeinen Form NHR₃⁺, die in Protonen und NR₃ zerfallen. Besonders bevorzugt ist das Ammonium-Kation (R = H), d.h. NH₄⁺.

Es ist jedoch auch möglich, größere, organische Ammonium-Kationen der Form NR₃R'⁺ zu wählen, bei denen sich der organische Rest R' durch Hofmann-Eliminierung derart zerlegt, dass aus dem Rest R' ein Olefin und Protonen entstehen. So ist z.B. bekannt, dass ein Butyl-Rest durch Hofmann-Eliminierung in ein Proton und in ein Buten (bevorzugt 1-Buten) gespalten werden kann. Das sich daraus bildende NR₃ sollte möglichst flüchtig sein.

Erfindungsgemäß besonders bevorzugt weisen die einsetzbaren Zeolithe eine Struktur auf, die aus den Strukturtypen Pentasil und MWW ausgewählt ist und ganz besonders bevorzugt aus den Strukturtypen MFI, MEL, Mischstrukturen aus MFI und MEL und MWW. Noch bevorzugter werden Zeolithe des Typs ZSM-5 oder MCM-22 eingesetzt. Die Bezeichnungen der Strukturtypen der Zeolithe entsprechen den Angaben in W. M. Meier, D. H. Olson und Ch. Baerlocher "Atlas of Zeolite Structure Types", Elsevier, 3. Auflage, Amsterdam 2001. Die Synthese der Zeolithe ist dem Fachmann bekannt und kann beispielsweise ausgehend von Alkalialuminat, Alkalisilikat und amorphem SiO2 unter hydrothermalen Bedingungen durchgeführt werden. Hierbei kann über organische Templat-Moleküle, über die Temperatur und weitere experimentelle Parameter die Art der gebildeten Kanalsysteme im Zeolithen gesteuert werden.

Die Katalysatorbelastung wird bei konstanter Katalysatormasse (limitiert durch den für die Anlage maximal möglichen Druckverlust) angegeben. Hierbei handelt es sich um eine reaktionstechnische Kenngröße, die den Massenstrom bzw. Molenstrom an Edukt in Bezug auf die Katalysatormasse angibt. Häufig wird auch das Volumen des Katalysatorbetts als Bezugsgröße verwendet (vgl. Gas Hourly Space Velocity oder Weight Hourly Space Velocity). In der Regel wird das Edukt mit einem Inertgas verdünnt auf die Schüttung aufgebracht. Diese Verdünnung wird aber nicht angegeben. Bei der vorliegenden Erfindung hat sich gezeigt, dass bei einer Eduktmenge von ∼50 mmol bei einer Dosierdauer zwischen 8 und 80 Minuten und einer Katalysatormasse zwischen 2 g und 5 g, eine Katalysatorbelastung von 2 bis 1000 mmol Amid/(g*h), bevorzugt von 3 bis 700 und besonders bevorzugt von 4 bis 500 mmol Amid/(g*h) vorliegt.

Der Druckverlust wird durch die Form der Katalysator-Partikel beeinflusst. Zeolithe können als Pulver eingesetzt werden. Oft ist dann aber der Druckverlust außerordentlich hoch. Um den Druckverlust einer Zeolith-Pulverschüttung zu verringern, kann diese Pulverschüttung durch Inertmaterial, z.B. aus Glas, als Formkörper etwas lockerer ausgeführt werden. Für den Einsatz als Katalysator werden die Zeolithe daher häufig auch als geformte Körper eingesetzt, um den Druckverlust der Schüttung niedrig zu halten. Zur Formgebung wird der Katalysator mit einem Bindemittel vermischt. Als Bindemittel eignen sich die üblichen, dem Fachmann bekannten Bindemittel wie Aluminiumoxid- und/oder Si-haltige Bindemittel. Besonders bevorzugt sind dabei Si-haltige Bindemittel; insbesondere eignen sich Tetraalkoxysilane, Polysiloxane und kolloidale SiO₂-Sole. Die Verwendung von geformten Zeolithen ist besonders bevorzugt. Möglich sind alle erdenklichen geometrischen Formen, die zu einer lockeren Packung der Schüttung führen. Bevorzugt sind insbesondere Kugeln, Zylinder und Sterne.

In manchen Fällen kann es vorteilhaft sein, die Zeolith-Formkörper noch mit anderen, inerten Formkörpern zu verdünnen, um eventuell infolge exothermer Reaktion auftretende Hotspots und die damit möglicherweise einhergehende Katalysatordesaktivierung zu reduzieren.

Zeolithe sind gerade in der Erdölindustrie häufig verwendete Katalysatoren. Sie dienen zur Isomerisierung bzw. zum Cracken von Kohlenwasserstoffen. Darüber hinaus können Zeolithe in unterschiedlichen Katalysebereichen als sogenannter Träger fungieren, auf die dann die eigentlich katalytisch aktiven Komponenten (in der Regel Metalle) aufgebracht werden. Zeolithe dienen darüber hinaus auch zur Reinigung / Trocknung (Molsiebe). Daher sind die Zeolithe leicht kommerziell verfügbar. Als Anbieter fungieren z.B. Clariant, BASF, Zeocem®, Grace, Zeolyst, Zeo Inc. usw. ExxonMobile verwendet auch viele Zeolithe.

Eine weitere Möglichkeit für geeignete Katalysatoren ist ein Stoffsystem, bei dem neben Aluminium, Silicium und Sauerstoff (vgl. Alumosilikate; Zeolithe) noch Phosphor involviert ist. Zur Erhöhung der Brönstedt-Acidität kann dem Zeolithen Phosphor beigemischt werden. Der Phosphor kann dem Zeolith durch jedes konventionelle Mittel zugefügt werden, wie Mischen des Zeolithen mit einer wässrigen Lösung einer Phosphorverbindung wie einem Phosphatsalz oder Phosphorsäure. Ammoniumhydrogenphosphate sind bevorzugte Phosphorquellen.

In einer weiteren Ausführungsform der Erfindung werden als Katalysatoren Aluminiumphosphate bzw. mit Phosphor-dotierten Aluminiumoxide eingesetzt. Derartige Materialien lassen sich entweder selbst herstellen oder sind kommerziell verfügbar. Ein möglicher Anbieter von Aluminiumphosphaten ist Clariant.

Aluminiumphosphat kann durch Reaktion von Aluminiumnitrat und Ammoniumphosphat in wäßriger Lösung erhalten werden, wobei im Besonderen eine wäßrige Lösung von Aluminiumnitrat und eine wäßrige Lösung von Ammoniumphosphat, die in einem molaren Phosphor-Aluminiumverhältnis von 0,5 bis 1,5 hergestellt werden, gemischt werden und diese Lösung nun vorzugsweise bei einem pH-Wert von 7 bis 9 durch Zugabe konzentrierter Ammoniumhydroxidlösung eingestellt wird. Nach Trocknen des erhaltenen Hydrogels wird dieses auf eine Temperatur von über 500° Celsius erhitzt (kalziniert). Das phosphor-dotierte Aluminiumoxid wird durch Mischen von Aluminiumoxid und Phosphorsäurelösung hergestellt (Imprägnierung). Diese Lösung enthält typischerweise 0,1 bis 30 Gewichtsprozent an Phosphorsäure im Gewichtsverhältnis von Phosphorsäure zu Aluminiumoxid. Das Aluminiumoxid hat eine spezifische Oberfläche größer als 1 m²/g, vorzugsweise mehr als 10 m²/g. Nach Entfernen des Wassers wird das Erzeugnis bei einer Temperatur von über 500° Celsius erhitzt, um den Phosphor auf dem Aluminiumoxid zu fixieren.

In einer weiteren Ausführungsform der Erfindung wird als Katalysator bzw. Katalysatorbestandteil Zirkondioxid verwendet. Zirkondioxid ist als keramischer Werkstoff bekannt. Zirkondioxid hat die Fähigkeit, bei höherer Temperatur Sauerstoffionen elektrolytisch zu leiten. Diese Eigenschaft macht man sich zunutze, um unterschiedliche Sauerstoffpartialdrücke z.B. zwischen Abgasen und Luft zu messen (Lambdasonde im Auto). Zirkondioxid kommt in unterschiedlichen Modifikationen vor, die sich bei höherer Temperaturen in einander umwandeln können: monoklin (bis 1173 °C) → tetragonal (2370 °C) → kubisch (2690 °C). Um das Zirkondioxid auch für Hochtemperaturanwendungen verfügbar zu haben, ohne dass sich durch Volumenänderung bei Phasenumwandlungen die mechanische Stabilität des Zirkondioxids ändert, wurde seitens der Keramik-Industrie intensiv daran gearbeitet, die Phasen durch Zugabe von anderen Oxiden zu stabilisieren. Es sind daher, mit anderen Elementen (z.B. Hf, Y, W) stabilisierte Zirkondioxide kommerziell verfügbar. Es ist darüber hinaus bekannt, dass Zirkondioxid in sulfatisiertes Zirkondioxid überführt werden kann (vgl. US-A 5 149 862), das sich durch eine höhere Acidität auszeichnet. Zirkondioxid ist beziehbar z.B. über die Firma Saint-Gobain Norpro oder über Ceramtec.

Denkbar als Katalysatoren sind weiterhin auch Heteropolysäuren (z.B. Wolframatophosphorsäure).

### Umsetzung zu den Fluoralkyltetrazolen der allgemeinen Formel (III) (Schritte 1.6 und 1.7 in Figur 1)

Im oben vorgestellten Verfahren liegen Verbindungen der allgemeinen Formel (I) in einem heißen Gas-Strom vor. In der Regel sind dem Gas-Strom inerte Verbindungen, wie z.B. Stickstoff, beigemischt. Die Abtrennung der Verbindungen der allgemeinen Formel (I) erfolgt durch Abkühlung in einer Kühl-/Kältefalle. Diese kann zur Unterstützung flüssiges oder festes Medium (Lösemittel / Aktivkohle oder ähnliches) enthalten, in dem Verbindungen der allgemeinen Formel (I) absorbiert oder adsorbiert werden können.

Die Isolierung der gewünschten Verbindungen der allgemeinen Formel (I) aus Lösemitteln kann beispielsweise durch Destillation, erfolgen.

Es ist jedoch nicht notwendig, Verbindungen der allgemeinen Formel (I) zu isolieren. Es ist sogar vorteilhaft, diese Verbindungen sofort (quasi *in situ*) weiter umzusetzen, insbesondere dann, wenn das erhaltene Nitril sehr giftig oder sehr reaktiv ist. Es ist bekannt, dass Nitrile mit vielen Reagenzien reagieren können. Die Reaktivität hängt typischerweise vom Rest R in R-C=N ab. Nitrile reagieren mit Laugen (OH⁻) zu den entsprechenden Carbonsäuren, mit Alkoholen und HCl lassen sich aus dem Nitril sogenannte Pinner-Salze erzeugen, Hieraus lassen sich in Folgeschritten Carbonsäureester, Orthoester oder protonierte Amidine bilden.

Es ist auch möglich, Verbindungen der allgemeinen Formel (I) zu primären Aminen zu hydrieren.

Eine weitere Möglichkeit der Funktionalisierung von Nitrilen ist die Umsetzung mit 1,3 dipolaren Verbindungen wie Nitriloxid, Azid oder Diazoalkan. Häufig führt eine derartige Umsetzung zu Heterozyklen, die bei der Herstellung von biologisch aktiven Verbindungen eine Rolle spielen. So reagiert ein Nitril mit einem Azid zu einem sogenannten Tetrazolat, d.h. Verbindungen der allgemeinen Formel (III).

Besonders bevorzugt ist erfindungsgemäß die *in situ* Erzeugung eines Nitrils gemäß Formel (I) und die sofortige Umsetzung (ohne Isolierung) mit einem Azid-Salz (vorzugsweise Natriumazid) in einem Lösemittel zu den Tetrazolverbindungen der allgemeinen Formel (III).

Dabei wird der produkthaltige Gasstrom aus der oben beschriebenen Gasphasen-Dehydratisierung über einen Wäscher (Schritt 1.6 in Figur 1) in eine Mischung (vorzugsweise in eine gerührte Suspension) aus Natriumazid in einem Lösungsmittel eingeleitet (1.7 in Figur 1). Vorzugsweise wird für diese Umsetzung ein aprotisch-polares Lösungsmittel wie z.B. Ketone wie Aceton, Lactone wie γ-Butyrolacton, Lactame wie N-Methyl-2-pyrrolidon, Nitrile wie Acetonitril, Nitroverbindungen wie Nitromethan, tertiäre Carbonsäureamide wie Dimethylformamid, Harnstoffderivate wie Tetramethylharnstoff oder Dimethylpropylenharnstoff (DMPU), Sulfoxide wie Dimethylsulfoxid (DMSO), Sulfone wie Sulfolan, Kohlensäureester wie Dimethylcarbonat oder Ethylencarbonat eingesetzt. Besonders bevorzugt wird Aceton oder Acetonitril als Lösungsmittel verwendet.

Der Wäscher (1.6 in Figur 1) enthält zur Abtrennung von Säurespuren vorzugsweise eine Base, um die Bildung explosionsfähiger Stickstoff-Wasserstoff-Säure (mögliche Entstehung durch Reaktion von Natriumazid mit Protonenquellen wie anorganische Säuren, Carbonsäuren, Alkoholen, Wasser) zu verhindern. Diese ist nicht nur äußerst reaktiv, sondern verringert auch die zur bestimmungsgemäßen Reaktion notwendigen Menge an Azid-Salz. Als Beispiel können wenig nukleophile Basen wie Pyridin oder substituierte Pyridine und substituierte oder unsubstituierte Chinoline verwendet werden. Bevorzugte Beispiele für geeignete Basen sind Pyridin, Picoline, Chinolin, Chinaldin und halogenierte Pyridine und besonders bevorzugt 3-Picolin.

Dem Natriumazidreaktor (1.7 in Figur 1) kann - zur weiteren Erhöhung der Prozesssicherheit - mit beispielsweise zwei Waschflaschen Natronlauge (20%ig) nachgeschaltet sein. Das Reaktorgas wird anschließend so stark verdünnt, dass es ohne Gefährdung der Abluft zugeführt werden kann.

Das oben beschriebene Verfahren wird vorzugsweise kontinuierliche durchgeführt. Die Strömungsgeschwindigkeiten werden vorzugsweise so gewählt, dass die jeweilige Verweilzeit im Reaktor im Bereich von 1.0 Sekunde bis 1 Minute vorzugsweise von 1.0 Sek. bis 10 Sek. liegt. Der Druck im Reaktor liegt typischerweise bei unter 1 bar, vorzugsweise zwischen 100 und 500 mbar und noch bevorzugter unter 300 mbar.

Die Isolierung der gewünschten Verbindungen der allgemeinen Formel (III) kann beispielsweise durch Filtration erfolgen. Hierzu sollte jedoch das in der Regel im Überschuss vorhandene und im Lösemittel nicht immer sehr gut lösliche Azid-Salz vorher abgetrennt worden sein. Alternativ kann mit einem etwas weniger polaren, aprotischen Lösemittel extrahiert werden.

Es ist denkbar, Verbindungen der allgemeinen Formel (III) seinerseits *in situ,* d.h. ohne Isolierung, weiter umzusetzen. Dies ist insbesondere dann vorteilhaft, wenn dadurch die Gefahr einer ungewollten (explosionsartigen) Zersetzung des Tetrazolats unter Stickstoff-Freisetzung weiter minimiert wird.

Die Menge an Natriumazid soll ausreichend groß sein, damit eine vollständige Umsetzung mit dem Nitril in einer technisch sinnvollen Zeit erfolgen kann. Ist der Überschuss an Azid zu groß, so muss das Salz, das sich potentiell unter Stickstoff-Freisetzung zersetzen kann, wieder abgetrennt werden.

Das molare Verhältnis Azid/Nitril liegt bevorzugt zwischen 1 und 10, bevorzugter zwischen 1 und 5 und besonders bevorzugt zwischen 1 und 2.

Die Menge an polarem, aprotischen Lösemittel, in der das Azid-Salz gelöst oder suspendiert ist, ist nicht kritisch. Typische Mischungen können bis zu 20 Gew.-% Azid enthalten.

Die vorliegende Erfindung wird anhand der nachfolgenden Beispiele näher erläutert, wobei die Beispiele nicht in die Erfindung einschränkende Weise zu interpretieren sind.

### Herstellungsbeispiele

Figur 2: Darstellung der Herstellung von Trifluormethyltetrazolat-Natrium ausgehend von Trifluoracetamid

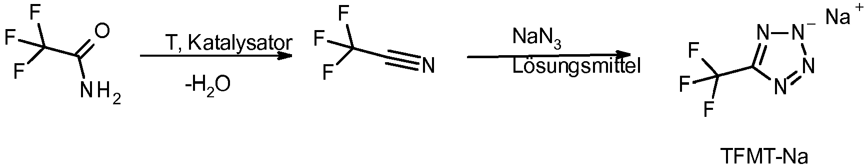

### Beispiel 1:

Der Reaktor wird mit 5.0 g Zeolith HCZP 55E (Clariant) befüllt und mit Stickstoff inertisiert. Nach Erreichen der Reaktionstemperatur von 500°C wird ein Stickstoffstrom von 55 ml/min eingestellt. Das geschmolzene Trifluoracetamid wird mit 0.5 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 5.4 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Lösung an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 1%.

### Beispiel 2:

Der Reaktor wird mit 5.0 g Zeolith HCZP 90E (Clariant) befüllt und mit Stickstoff inertisiert. Nach Erreichen der Reaktionstemperatur von 350°C wird ein Stickstoffstrom von 55 ml/min eingestellt. Das geschmolzene Trifluoracetamid wird mit 0.5 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 5.4 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Lösung an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 3%.

### Beispiel 3:

Der Reaktor wird mit 2.0 g Zeolith HCZP 27E (Clariant) befüllt und mit Stickstoff inertisiert. Nach Erreichen der Reaktionstemperatur von 350°C wird ein Stickstoffstrom von 99 ml/min eingestellt.

Das geschmolzene Trifluoracetamid wird mit 0.05 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 2.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 18%.

### Beispiel 4:

Der Reaktor wird mit 5.0 g Zeolith HCZP 55E (Clariant) befüllt und mit Stickstoff inertisiert. Nach Erreichen der Reaktionstemperatur von 400°C wird ein Stickstoffstrom von 99 ml/min eingestellt. Das geschmolzene Trifluoracetamid wird mit 0.5 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 2.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 13%.

### Beispiel 5:

Der Reaktor wird mit 5.0 g dotiertes Zirkonoxid SZ69157 (Saint-Gobain) befüllt und mit Stickstoff inertisiert. Nach Erreichen der Reaktionstemperatur von 400°C wird ein Stickstoffstrom von 99 ml/min eingestellt. Das geschmolzene Trifluoracetamid wird mit 0.14 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 2.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 18%.

### Beispiel 6:

Der Reaktor wird mit 1.2 g dotiertem Aluminiumphosphat HCZA (Clariant) als Pulver verdünnt mit 9 g Inertmaterial (Glasperlen) befüllt und mit Stickstoff inertisiert. Nach Erreichen der Reaktionstemperatur von 400°C wird ein Stickstoffstrom von 99 ml/min eingestellt. Das geschmolzene Trifluoracetamid wird mit 0.14 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 2.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 4%.

### Beispiel 7:

Der Reaktor wird mit 2.0 g Zeolith HCZP 55E (Clariant) befüllt und mit Stickstoff inertisiert. Nach Erreichen der Reaktionstemperatur von 300°C wird ein Stickstoffstrom von 150 ml/min eingestellt. Das geschmolzene Trifluoracetamid wird mit 0.05 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 1.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 11%.

### Beispiel 8:

Der Reaktor wird mit 5.0 g Zeolith HCZP 55E (Clariant) befüllt und mit Stickstoff inertisiert. Nach Erreichen der Reaktionstemperatur von 350°C wird ein Stickstoffstrom von 150 ml/min eingestellt. Das geschmolzene Trifluoracetamid wird mit 0.05 ml/min in den vorgeschalteten Verdampfer (T=250°C) geleitet und von dort ebenfalls in den Reaktor. Die mittlere Verweilzeit in der Reaktionszone ergibt sich aus dem Reaktoraufbau und beträgt 1.9 sec. Der Produktgasstrom wird zunächst durch einen auf +4°C gekühlten Intensivkühler geleitet und anschließend über einen mit 3-Picolin befüllten Wäscher in eine Suspension aus 10 wt% Natriumazid in Aceton eingeleitet. Die resultierende Menge an Trifluormethyltetrazolat-Natrium (TFMT-Na) in Aceton wird via ¹⁹F-NMR unter Verwendung eines internen Standards analysiert. Die Ausbeute an TFMT-Na bezogen auf die eingesetzte Menge Trifluoracetamid beträgt 27%.

## Patentansprüche

1. Verfahren zum Herstellen von Fluoralkylnitrilen der allgemeinen Formel (I) durch katalytische Gasphasen-Dehydratisierung, 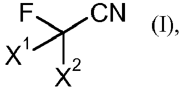 in welcher
X¹ und X² unabhängig voneinander für ein Halogen, Wasserstoff, Methyl stehen,
**dadurch gekennzeichnet, dass** man
fluorierte Carbonsäureamide der Formel (II) 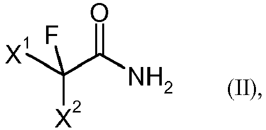 in welcher X¹ und X² die oben genannten Bedeutungen haben, in die Gasphase überführt und in Gegenwart von Katalysatoren aus der Gruppe von Zeolithen, Aluminiumphosphat, Zirkondioxid und Heteropolysäuren
umsetzt.

2. Verfahren nach Anspruch 1, wobei X¹ und X² jeweils unabhängig voneinander für Fluor, Chlor, Wasserstoff, Methyl und bevorzugt für Fluor stehen.

3. Verfahren nach einem der Ansprüche 1 bis 2, wobei der Katalysator ein Zeolith ist.

4. Verfahren nach Anspruch 3, wobei der Zeolith-Katalysator in Protonen-Form vorliegt und das molare Verhältnis von SiO₂ zu AlO₂ 10-120 beträgt.

5. Verfahren nach Anspruch 3 oder 4, wobei der Zeolith eine Struktur aufweist, die ausgewählt ist aus der Gruppe von Pentasil und MWW.

6. Verfahren gemäß einem der Ansprüche 1 bis 5, wobei fluorierte Carbonsäureamide der Formel (II) bei Temperaturen von 200-500°C in Gegenwart der genannten Katalysatoren vorzugsweise in einem Reaktor zu den Fluoralkylnitrilen der allgemeinen Formel (I) umgesetzt werden.

7. Verfahren nach Anspruch 6, wobei die Reaktion bei einem Druck von weniger als 1 bar stattfindet.

8. Verfahren gemäß einem der Ansprüche 6 bis 7, wobei das Verfahren kontinuierlich durchgeführt wird und die Strömungsgeschwindigkeiten so gewählt sind, dass die jeweilige Verweilzeit im Reaktor im Bereich von 1.0 Sekunde bis 1 Minute liegt.

9. Verfahren gemäß einem der Ansprüche 1 bis 8, **dadurch gekennzeichnet, dass** man die erhaltenen Fluoralkylnitrile der allgemeinen Formel (I), 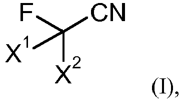 in Gegenwart eines Lösungsmittels mit Natriumazid zu den entsprechenden Fluoralkyltetrazolen der allgemeinen Formel (III) 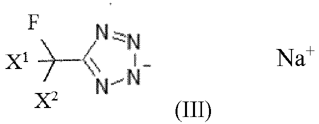 in welcher X¹ und X² die oben genannten Bedeutungen haben, umsetzt.

10. Verfahren nach Anspruch 9, wobei man die Fluoralkylnitrile der allgemeinen Formel (I) aus einem Verfahren nach einem der Ansprüche 1 bis 8 erhält und nach Kühlung und Waschung einer Mischung aus Lösungsmittel und Natriumazid zuführt.

## Claims

1. Process for the preparation of fluoroalkylnitriles of the general formula (I) by catalytic gas-phase dehydration, 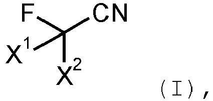 in which
X¹ and X² are, independently of each other, a halogen, hydrogen or methyl,
**characterized in that**
fluorinated carboxamides of the formula (II) 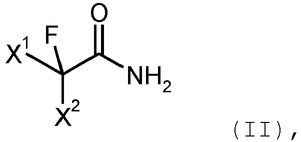 in which X¹ and X² have the abovementioned meanings, are converted into the gas phase and are reacted in the presence of catalysts from the group of zeolites, aluminium phosphate, zirconium dioxide and heteropolyacids.

2. Process according to Claim 1, in which X¹ and X² are each time, independently of each other, fluorine, chlorine, hydrogen or methyl and preferably fluorine.

3. Process according to either of Claims 1 and 2, in which the catalyst is a zeolite.

4. Process according to Claim 3, in which the zeolite catalyst is present in proton form and the molar ratio of SiO₂ to AlO₂ is 10-120.

5. Process according to Claim 3 or 4, in which the zeolite exhibits a structure which is chosen from the group of pentasil and MWW.

6. Process according to one of Claims 1 to 5, in which fluorinated carboxamides of the formula (II) are converted at temperatures of 200-500°C in the presence of the catalysts mentioned, preferably in a reactor, to give the fluoroalkylnitriles of the general formula (I).

7. Process according to Claim 6, in which the reaction takes place at a pressure of less than 1 bar.

8. Process according to either of Claims 6 and 7, in which the process is carried out continuously and the flow velocities are chosen so that the respective residence time in the reactor is in the range from 1.0 second to 1 minute.

9. Process according to one of Claims 1 to 8, **characterized in that** the fluoroalkylnitriles obtained of the general formula (I), 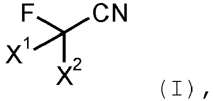 are reacted in the presence of a solvent with sodium azide to give the corresponding fluoroalkyltetrazoles of the general formula (III) 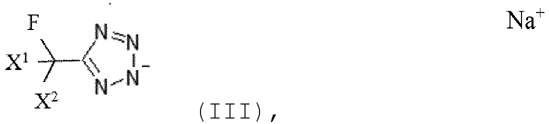 in which X¹ and X² have the abovementioned meanings.

10. Process according to Claim 9, in which the fluoroalkylnitriles of the general formula (I) are obtained from a process according to one of Claims 1 to 8 and, after cooling and scrubbing, are led to a mixture of solvent and sodium azide.

## Revendications

1. Procédé pour la préparation de fluoroalkylnitriles de formule générale (I) par déshydratation catalytique en phase gazeuse, 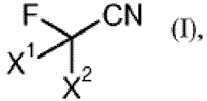 dans laquelle
X¹ et X² représentent indépendamment l'un de l'autre un halogène, hydrogène, méthyle,
**caractérisé en ce qu'**on transfère en phase gazeuse des amides d'acides carboxyliques fluorés de formule (II) 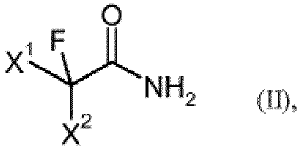 dans laquelle X¹ et X² possèdent les significations mentionnées ci-dessus, et on les transforme en présence de catalyseurs du groupe comprenant des zéolithes, le phosphate d'aluminium, le dioxyde de zirconium et des hétéropolyacides.

2. Procédé selon la revendication 1, X¹ et X² représentant indépendamment l'un de l'autre fluor, chlore, hydrogène, méthyle et préférablement représentant fluor.

3. Procédé selon l'une quelconque des revendications 1 et 2, le catalyseur étant une zéolithe.

4. Procédé selon la revendication 3, le catalyseur de type zéolithe étant présent sous forme protonée et le rapport molaire de SiO₂ sur AlO₂ étant de 10 à 120.

5. Procédé selon la revendication 3 ou 4, la zéolithe présentant une structure qui est choisie dans le groupe comprenant Pentasil et MWW.

6. Procédé selon l'une quelconque des revendications 1 à 5, des amides d'acides carboxyliques fluorés de formule (II) étant transformés à des températures de 200 à 500 °C en présence des catalyseurs mentionnés de préférence dans un réacteur pour donner les fluoroalkylnitriles de formule générale (I).

7. Procédé selon la revendication 6, la réaction ayant lieu à une pression inférieure à 1 bar.

8. Procédé selon l'une quelconque des revendications 6 et 7, le procédé étant mis en œuvre de manière continue et les vitesses d'écoulement étant choisies de telle sorte que le temps de séjour respectif dans le réacteur se situe dans la plage de 1,0 seconde à 1 minute.

9. Procédé selon l'une quelconque des revendications 1 à 8, **caractérisé en ce qu'**on transforme les fluoroalkylnitriles de formule générale (I) obtenus, 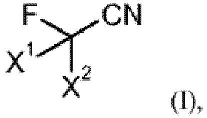 en présence d'un solvant avec de l'azoture de sodium pour donner les fluoroalkyltétrazoles correspondants de formule générale (III) 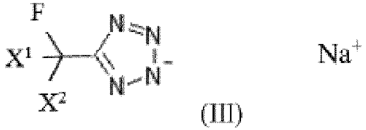 dans laquelle X¹ et X² possèdent les significations mentionnées ci-dessus.

10. Procédé selon la revendication 9, dans lequel on obtient les fluoroalkylnitriles de formule générale (I) par un procédé selon l'une quelconque des revendications 1 à 8 et après refroidissement et lavage, on les introduit dans un mélange de solvant et d'azoture de sodium.
